# EUROPÄISCHE PATENTSCHRIFT

(11) **EP 2 241 348 B1**
(45) Veröffentlichungstag und Bekanntmachung des Hinweises auf die Patenterteilung: **06.05.2015**
(21) Anmeldenummer: 10156625.5
(22) Anmeldetag: 16.03.2010
(51) Int. Cl.: A61N 1/365, A61N 1/362, A61B 5/0215, A61B 5/053, A61B 5/107

(54) **Herzmonitor**
Heart monitor
Dispositif de surveillance cardiaque

(30) Priorität: 15.04.2009 DE 102009002397
(43) Veröffentlichungstag der Anmeldung: 20.10.2010
(73) Patentinhaber: Biotronik CRM Patent AG, 6341 Baar (CH)
(72) Erfinder: Vollkron, Michael, 3021 Pressbaum (AT); Czygan, Gerald, 91054 Buckenhof (DE); Krämer, Thomas, 90425 Nürnberg (DE); Lippert, Michael, 91522 Ansbach (DE); Skerl, Olaf, 18209 Bad Doberan (DE)
(74) Vertreter: Lindner-Vogt, Karin L.

(56) Entgegenhaltungen:
- WO-A2-2006/107922
- US-A1- 2007 066 898
- US-A1- 2007 129 765
- US-B1- 7 286 875

## Beschreibung

Die Erfindung betrifft einen Herzmonitor, der wenigstens mit einem Sensor für Druck- und Volumendaten oder Ersatzgrößen eines Herzens verbunden ist oder verbunden werden kann.

Ein solcher Herzmonitor ist beispielsweise aus US 2004/0030356 bekannt. Die mit einem derartigen Herzmonitor gewonnenen Druck- und Volumendaten können beispielsweise - ähnlich wie in US 2004/0030356 beschrieben - zur Optimierung der Stimulationsparameter eines ggf. implantierbaren Herzstimulators verwandt werden.

Die US2007/066898 beschreibt ein Ultraschallsystem mit einem Drucksensor zur Darstellung von Ultraschallsignalen und Druckdaten.

Die US7,286,875 hingegen beschreibt ein implantierbaren Herzstimulator zur Bestimmung der ventrikulären Kontraktion.

Die US2007/0129765 beschreibt ein Implantat und eine Methode zur Messung von Druck-Volumenkurven eines Herzens unter Verwendung eines Geräuschsensors.

Die WO2006/107922 offenbart eine Methode zur Optimierung eines biventrikulären Schrittmachers.

Der in dieser Anmeldung beschriebene Herzmonitor kann beispielsweise Bestandteil eines implantierbaren Impulsgenerators, wie beispielsweise eines Herzschrittmachers oder eines implantierbaren Cardioverter/Defibrillators (ICD), sein.

Aufgabe der Erfindung ist es, einen verbesserten Herzmonitor zu schaffen.

Erfindungsgemäß wird diese Aufgabe durch einen Herzmonitor der eingangs genannten Art gelöst, der eine Auswerteeinheit zur Verarbeitung wenigstens eines den zeitlichen Verlauf von Druck- und Volumendaten oder Ersatzgrößen des Herzens widerspiegelnden Eingangssignals aufweist, die derart ausgebildet ist, dass die Auswerteeinheit eine Segmentierung des Eingangssignals entsprechend einzelner abgeschlossener Herzzyklen vornimmt und so gewonnene Segmente des Eingangssignals jeweils daraufhin überprüft, ob ein jeweiliges Segment des Eingangssignals ein pV-Diagramm repräsentiert, das vorgegebenen Qualitätsbedingungen bezüglich Umlaufrichtung, Morphologie sowie Abstand zwischen Anfangs und Endwert entspricht. Die Auswerteeinheit ist dazu ausgebildet, aufeinander folgende Signalwertepaare des Eingangssignals hinsichtlich einer Umlaufrichtung eines durch die Signalwertepaare repräsentierten pV-Diagramms auszuwerten und die Umlaufrichtung eines jeweiligen durch Signalwertepaare repräsentierten pV-Diagramms dazu zu verwenden, ungültige Signalwertepaare (also solche Signalwertepaare, die eine der allgemeinen Umlaufrichtung entgegen gerichtete Umlaufrichtung ergeben) zu erkennen und ggf. zu korrigieren. Dies kann beispielsweise dadurch geschehen, dass die Auswerteeinheit ausgebildet ist, solche Signalwertepaare zu verwerfen, die eine abweichende Umlaufrichtung haben.

Unter einem pV-Diagramm versteht man die zweidimensionale Darstellung von Druck- und Volumenmessdaten während eines Herzzyklus, wobei die einzelnen Signalwertepaare gemäß ihrer zeitlichen Reihenfolge verbunden sind.

PV-Diagramme sind grundsätzlich bekannt und können zur Bestimmung klinisch relevanter Parameter herangezogen werden. Beispielsweise kann die Kontraktilität der Ventrikel, der myokardiale Sauerstoffbedarf, eine Abschätzung der Effizienz des Herzens sowie die Abstimmung von Kontraktilität des Herzens und peripherer Last bestimmt werden. Diese Parameter sind bisher nur während eines Klinikaufenthaltes verfügbar. Eine kontinuierliche Nutzung ist bisher aus diesem Grund nicht möglich. Der erfindungsgemäße Herzmonitor bietet den Vorteil, eine derartige kontinuierliche Nutzung der pV-Diagramme zu ermöglichen und löst in diesem Zusammenhang weiterhin das Problem, dass nach Möglichkeit nur solche pV-Diagramme einer weiteren Auswertung unterzogen werden sollten, die auf plausible und stabile Messwerte zurückgehen. Daher prüft der erfindungsgemäße Herzmonitor, ob das einen jeweiligen Herzzyklus repräsentierende pV-Diagramm stabil ist und den vorgegebenen Qualitätsbedingungen entspricht. Geringe Abweichungen sind dabei zulässig, da diese beispielsweise auch Folge von schnellen Autoregulationsmechanismen und/oder Laständerungen sein können.

Vorzugsweise kann die Auswerteeinheit ausgebildet sein, die Umlaufrichtung eines jeweils durch die Signalwertepaare repräsentierten pV-Diagramms durch Auswerten der Drehung zweidimensionaler Vektoren zu bestimmen, indem sie ein zweidimensionales Koordinatensystem mit einem Ursprung im Zentrum eines jeweiligen pV-Diagramms generiert und für jedes Signalwertepaar einen Vektor vom Mittelpunkt des Koordinatensystems zu einem jeweiligen Signalwertepaar bestimmt, um die Umlaufrichtung aufeinander folgender Signalwertepaare zu bestimmen. Die Umlaufrichtung eines durch die Signalwertepaare repräsentierten pV-Diagramms ist deshalb ein geeignetes Kriterium zur Überprüfung der Plausibilität der Signalwertepaare, da ein tatsächliches pV-Diagramm immer nur eine feste Umlaufrichtung hat. Sollten die Messwerte ein pV-Diagramm repräsentieren, das einen Wechsel in der Umlaufrichtung aufweist, kann dies nur auf einen fehlerhaften Signalwert zurückgehen.

Eine weitere bevorzugte Ausführungsvariante eines Herzmonitors bezieht sich auf Herzmonitore mit einem Markersignaleingang für Markersignale, die definierte Ereignisse innerhalb eines jeweiligen Herzzyklus einschließlich des Zeitpunktes ihres Auftretens repräsentieren. Hierbei ist die Auswerteeinheit vorzugsweise so ausgebildet, dass sie eine Segmentierung des Eingangssignals anhand über den Markersignaleingang zu empfangender Markersignale vornimmt.

Zusätzlich oder alternativ kann die Auswerteeinheit auch ausgebildet sein, eine Segmentierung des Eingangssignals anhand morphologischer Merkmale der durch die Signalwertepaare des Eingangssignals repräsentierten pV-Diagramme vorzunehmen.

Eine weitere Alternative besteht darin, dass der Herzmonitor einen EKG-Signaleingang für ein intrakardiales EKG-Signal oder Oberflächen EKG aufweist, wobei die Auswerteeinheit ausgebildet ist, eine Segmentierung des Eingangssignals anhand eines über den EKG-Signaleingang zu empfangenden intrakardialen EKG-Signals vorzunehmen.

Weiterhin kann die Auswerteeinheit ausgebildet sein, eine Approximation der durch die Signalwertepaare repräsentierten pV-Diagramme durch Ersatzfunktionen, wie Ellipsen, Rechtecke, Trapeze oder dergleichen, vorzunehmen. Dabei kann die Auswahl einer möglichst günstigen Geometrie entweder manuell erfolgen und somit manuell vorgegeben werden oder auch automatisch durch die Auswerteeinheit erfolgen.

Hinsichtlich einer Korrektur möglicherweise fehlerhafter Signalwertepaare ist es bevorzugt, wenn die Auswerteeinheit ausgebildet ist, Kreuzungspunkte der durch die Signalwertepaare repräsentierten pV-Diagramme, die sich durch die Verbindung aufeinander folgender Signalwertepaare ergeben, automatisch zu erkennen und zu korrigieren. Solche Kreuzungspunkte entstehen beispielsweise durch Messrauschen oder Mess-Ungenauigkeiten und kommen in einem tatsächlichen pV-Diagramm nicht vor.

Im Hinblick auf die weitere Auswertung wie zuvor beschrieben korrigierter pV-Diagramme ist die Auswerteeinheit ausgebildet, ein jeweils korrigiertes pV-Diagramm mit dem jeweils entsprechenden, ursprünglichen unkorrigierten pV-Diagramm zur Ableitung von Qualitätsmerkmalen zu vergleichen. Hierbei ist es bevorzugt, wenn die Auswerteeinheit ausgebildet ist, den Vergleich anhand von Kriterien, wie zum Beispiel der Anzahl der korrigierten Signalwertepaare, der Unterschiede in der Morphologie der repräsentierten pV-Diagramme, der Flächendifferenzen zwischen den repräsentierten pV-Diagrammen, Schwerpunktverschiebungen zwischen den pV-Diagrammen, der Entfernung zwischen End- und Startpunkt eines jeweiligen pV-Diagramms oder dergleichen durchzuführen und in Abhängigkeit eines jeweiligen Vergleichsergebnisses mit vorgegebenen Qualitätskriterien zu vergleichen und zu entscheiden, ob sich ein jeweiliges korrigiertes pV-Diagramm für eine weitere Auswertung eignet oder nicht.

Außerdem kann die Auswerteeinheit ausgebildet sein, alle ein jeweiliges pV-Diagramm repräsentierenden Signalwertepaare durch eine Hüllkurve zu umschließen. Hierbei kann die Auswerteeinheit ausgebildet sein, eine jeweils größtmögliche Hüllkurve zu bestimmen, die keinen, oder die kleinstmögliche, die die meisten oder alle Signalwertepaare eines ein jeweiliges pV-Diagramm repräsentierenden Signalsegmentes umhüllt.

Alternativ kann die Auswerteeinheit auch ausgebildet sein, eine ein jeweiliges pV-Diagramm repräsentierende Folge von Signalwertepaaren durch eine oder mehrere Parameter-optimierte Funktionen zu approximieren.

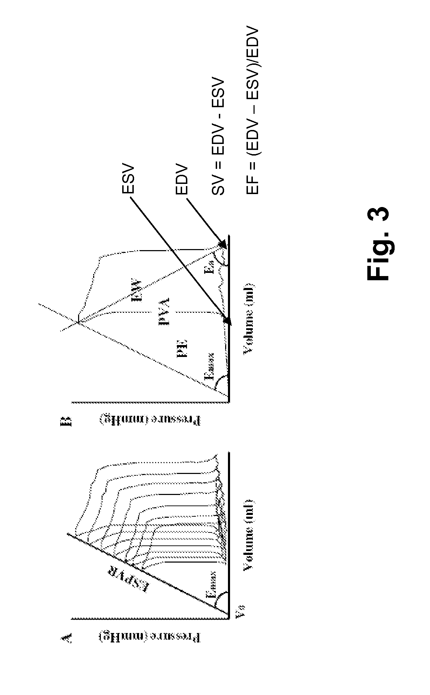

Die bis hierhin beschriebenen vorausgewerteten Signalwertepaare - genauer gesagt: Folgen von Signalwertepaaren des Eingangssignals, die ein jeweiliges pV-Diagramm repräsentieren - können anschließend weiter ausgewertet werden. Hierzu ist die Auswerteeinheit vorzugsweise ausgebildet, aus einem jeweiligen durch Signalwertepaare repräsentierten pV-Diagramm (linker oder rechter Ventrikel) jeweils einen Wert für einen oder mehrere der folgenden Parameter abzuleiten:
- Kontraktilitätsindex (Emax = ESP/(ESV - Vo)): Emax entspricht dem Anstieg jener Geraden die durch Vo gehend die pV-Kurve am linken oberen Rand gerade noch berührt (siehe Fig. 3). Der End-systolische Druck ESP ist jener Druck im Ventrikel der sich am Ende der Auswurfphase einstellt.
- End-diastolisches Volumen (EDV): Das maximale Blutvolumen im Ventrikel, gemessen am Ende der Füllphase jedes Herzschlages
- End-systolisches Volumen (ESV): Das minimale Blutvolumen im Ventrikel, gemessen am Ende der Austreibungsphase
- Schlagvolumen (SV): Das Schlagvolumen eines einzelnen Herzschlages (SV = EDV - ESV)
- Ejektionsfraktion (EF = SV/EDV)
- Effektive arterielle Elastanz (Ea): Die effektive Elastanz des an den linken Ventrikel anschließenden arteriellen Systems (Ea = ESP/SV)
- Externe Arbeit (EW): Jener Anteil der Herzarbeit, die direkt in mechanische Energie umgewandelt wird. Bestimmt wird dieser Parameter durch die Berechnung der Fläche innerhalb der PV-Kurve;
- Potentielle Energie (PE): Jener Anteil der Herzarbeit, die nicht in mechanische Energie umgewandelt werden kann. PE bezeichnet eine Fläche, die an die PV-Kurve linksseitig anschließt. Sie verläuft ausgehend vom Residualvolumen (Vo) über jene Gerade die Vo mit dem End-systolischen Druck ESP verbindet, weiter dem Verlauf der isovolumetrischen Relaxation folgend, bis zum Öffnen der Einstromklappe und wieder zurück zu Vo (siehe Fig. 3). Möglichkeiten zur direkten Bestimmung von Vo sind durch die Veränderung von Elektrostimulationsparametern wie AV und VV Verzögerung, Herzrate aber auch durch die künstliche Erzeugung von Extrasystolen gegeben da die verschiedenen End-systolischen Drücke auf einer Geraden liegen, deren Schnittpunkt mit der Volumenachse, Vo entspricht. Bei der Verwendung von Ersatzgrößen werden diese Merkmale entsprechend transformiert. Vo wird zu diesem Zweck geschätzt oder aus echokardiographischen Vermessungen bestimmt.
- Druck-Volumen Fläche (PVA): Der Gesamte Energiebedarf des Herzens während eines Schlages (PVA = PE + EW). Diese Größe kann zur Bestimmung des myokardialen Sauerstoffbedarfs verwendet werden;
- EW/PE: Die Effizienz des Herzens kann aus dem Verhältnis von EW und PE bestimmt werden. Je größer dieses Verhältnis ist umso höher ist der Anteil jener Energie die vom Herzen in mechanische Arbeit umgesetzt werden kann. Als Alternative wird für den gleichen Zweck auch EW/PVA, also das Verhältnis von geleisteter mechanischer Arbeit zum Gesamten Energiebedarf des Herzens, verwendet;
- Arteriell-ventrikuläre-Kopplung (AVC): AVC = Ea/Emax = (ESV-Vo)/ SV. Dieser Parameter kann auch ohne Messung des linksventrikulären Drucks bestimmt werden.
- Preload-Recruitable-Stroke_Work (PRSW): Beschreibt das Verhältnis von EW und EDV und dient als Vor- und Nachlast unabhängiger Parameter für die myokardiale Kontraktilität (alternative zu Emax).

Für die Berechnung von Emax, PE, PVA sowie AVC ist eine Bestimmung des Residualvolumens Vo erforderlich. Das Residualvolumen ist jenes Blutvolumen bei dem eine Kontraktion des Ventrikels zu einem End-systolischen Druck von 0 mmHg führen würde. Dieses kann durch bildgebende Verfahren wie bspw. Echokardiographie erfolgen, oder für den Fall, dass nur Änderungen betrachtet werden, auch auf einen hinreichend niedrigen festen Wert gesetzt werden. Da Vo im Allgemeinen klein gegenüber ESV ist.

Bevorzugt wird auch, dass Werte abgeleiteter Parameter über die Zeit ermittelt und gespeichert werden. Diese Verläufe können zur Beobachtung des Patienten oder als Eingangsgröße für Prädiktionsalgorithmen verwendet werden.

Gemäß bevorzugter Ausführungsvarianten der Erfindung kann der Herzmonitor mit einem Herzstimulator verbunden sein und ausgebildet sein, die Abgabe von Stimulationsimpulsen durch den Herzstimulator zu beeinflussen. Hierbei ist die Auswerteeinheit vorzugsweise dazu ausgebildet, im Rahmen der weiteren Auswertung von z. B. transienten Kontraktilitätsänderungen (Emax) durch eine Variation von Stimulationsparametern, wie die atrioventrikuläre Verzögerungszeit (AVD), die interventrikuläre Verzögerungszeit (VVD), die Stimulationsfrequenz oder dergleichen anzuregen, um ein jeweiliges Residualvolumen Vo bestimmen zu können.

Alternativ hierzu kann die Auswerteeinheit auch dazu ausgebildet sein, das Residualvolumen Vo auch ohne Variation von Stimulationsparametern ganz ohne eine Stimulation des jeweiligen Herzens dadurch automatisch vorzunehmen, dass die Auswerteeinheit transiente Kontraktilitätsänderung zur Aktualisierung des Wertes für das Residualvolumen Vo nutzt.

Gemäß einer ebenfalls bevorzugten Ausführungsvariante ist der Herzmonitor mit einem Herzstimulator verbunden und ausgebildet, die Abgabe von Stimulationsimpulsen durch den Herzstimulator zu beeinflussen, wobei die Auswerteeinheit weiter ausgebildet ist, eine der vorstehend genannten abgeleiteten Größen, wie Emax, EDV, ESV etc. zur gleichzeitigen Optimierung von einer atrioventrikulären Überleitungszeit (AVD) und oder interventrikulären Überleitungszeit (VVD) und oder Stimulationsfrequenz zu verwenden, indem die atrioventrikuläre Übergangszeit (AVD) und/oder die interventrikuläre Übergangszeit (VVD) und/oder die Stimulationsfrequenz so lange variiert werden, bis sich ein globales Optimum ergibt.

Hinsichtlich der Aufnahme der Messwerte, die schließlich - ggf. nach entsprechender Verstärkung etc. - die Signalwerte des Eingangssignals ergeben, ist der Signalmonitor vorzugsweise mit entsprechenden Messsensoren verbunden. Diese Messsensoren können beispielsweise Drucksensoren oder Kraftsensoren sein, aber auch Abstandssensoren, wie nachfolgend näher ausgeführt ist.

Verwendete Drucksensoren können beispielsweise in typischen Elektrodenleitungen eines Herzstimulators angeordnet sein und damit eine Druckmessung direkt in einer Herzkammer ermöglichen. Zur Bestimmung des Drucks in der linken Herzkammer kann der Druck während der Diastole durch eine Messung des Drucks im linken Vorhof und während der Systole durch eine Messung des arteriellen Drucks in der Aorta bestimmt werden.

Ein Drucksensor kann auch in einer Coronarsinus-Elektrodenleitung integriert sein. Während einer Systole steigt der Druck in den Coronargefäßen infolge der Kontraktion des Herzmuskels an. Durch Messung dieser Druckänderung, beispielsweise durch einen Drucksensor in einer Coronarsinus-Elektrodenleitung, kann eine Abschätzung des Ventrikeldrucks erfolgen.

Mit Hilfe von Kraftsensoren kann beispielsweise eine Kraftmessung zwischen zwei fixen Punkten an der Herzwand erfolgen. Dies ist bei Patienten anwendbar, die Myokardbereiche mit nicht kontrahierendem Gewebe aufweisen.

Zusätzlich oder alternativ kann auch eine mechanische Fixiereinrichtung zur Positionierung einer Coronarsinus-Elektrode genutzt werden, um die Kraftentwicklung während der Systole und damit indirekt den Druckverlauf im Inneren der Herzkammer zu erfassen.

Als weitere Alternative können lokale, direkt ins Myokard eingebrachte Kraft- oder DruckSensoren vorgesehen sein, die zur Bestimmung der Druckentwicklung im Inneren des Ventrikels verwendet werden können.

Hinsichtlich des Einsatzes von Abstandssensoren besteht die Möglichkeit, eine Abstandsmessung zwischen zwei fixen Punkten an der Herzwand vorzunehmen. Diese Methode ist bei Patienten anwendbar, die Myokardbereiche mit nicht kontrahierendem Gewebe aufweisen.

Hinsichtlich des Einsatzes von Drucksensoren, die mit dem Herzmonitor verbunden sind, besteht auch die Möglichkeit, diese im Septum zwischen den Herzkammern anzuordnen. Diese können so ausgebildet sein, dass sie den Druck oder eine Kraftentwicklung im Septum erfassen oder alternativ transseptal direkt den Ventrikeldruck messen.

Auch kann der Herzmonitor mit einem Druckmesskatheter verbunden sein, mit dem langzeitstabile Druckmessungen im Ventrikel oder im Endokard durchgeführt werden können, um so Messwerte zu gewinnen, die dem Herzmonitor übermittelt werden.

Alternativ können auch solche Drucksensoren im Inneren eines oder beider Ventrikel oder im Endokard angebracht sein, die eine Sendeeinheit enthalten und ihre Messwerte drahtlos an den Herzmonitor übertragen.

Alternativ oder zusätzlich kann der Herzmonitor auch ausgebildet sein, ventrikuläre Drücke aus Ersatzdrücken zu ermitteln, die mit Drucksensoren an ventrikulären Elektrodenleitungen oder mit Drucksensoren, die innen oder außen an der Herzwand befestigt sind oder minimalinvasiv in das Perikard eingebracht wurden gemessen werden.

Alternativ oder zusätzlich kann der Herzmonitor auch ausgebildet sein, ventrikuläre Volumina aus Ersatzgrößen (Impedanzmessung, Ultraschall-Laufzeitmessung, Dämpfung von RF-Signalen, Dämpfung von Schallsignalen) zu ermitteln, die mit Abstandssensoren gemessen werden, die an ventrikulären Elektrodenleitungen oder innen oder außen an der Herzwand befestigt sind oder minimalinvasiv in das Perikard eingebracht wurden.

Der Herzmonitor kann alternativ auch ausgebildet sein, einen ventrikulären Druck durch Auswerten eines intrakardialen Impedanzverlaufs (z. B. durch Differenziation) abzuleiten. Die hierzu erforderliche Bestimmung des intrakardialen Impedanzverlaufs ist grundsätzlich bekannt und andernorts ausführlich beschrieben.

Der Herzmonitor kann grundsätzlich als selbständiges Gerät, beispielsweise als selbständiges Implantat, ausgeführt sein. Er kann außerdem Bestandteil eines implantierbaren medizinischen Gerätes, wie beispielsweise eines Herzschrittmachers oder eines Cardioverters/Defibrillators (ICD) sein. Dabei kann das implantierbare medizinische Gerät auch ein Implantat im näheren Sinne umfassen sowie ein externes Gerät, das telemetrisch mit dem Implantat im engeren Sinne verbunden ist. Der Herzmonitor der hier beschriebenen Art und insbesondere dessen Auswerteeinheit können dabei ganz oder in Teilen sowohl Bestandteil des Implantats im engeren Sinne oder des externen Geräts oder beider oder weiterer Komponenten des medizinischen Geräts sein.

Die Erfindung soll nun anhand eines Ausführungsbeispiels mit Bezug auf die Figuren näher erläutert werden. Von diesen zeigt:
- Fig. 1:: Ein medizinisches Gerät umfassend ein Implantat und ein externes Gerät sowie ein zentrales Servicecenter;
- Fig. 2:: einen Herzmonitor als medizinisches Gerät bzw. Bestandteil eines medizinischen Gerätes; und
- Fig. 3:: Diagramme zur Erläuterung eines Druck-Volumen-Verlaufs im Inneren eines Ventrikels sowie hieraus abzuleitender Größen.

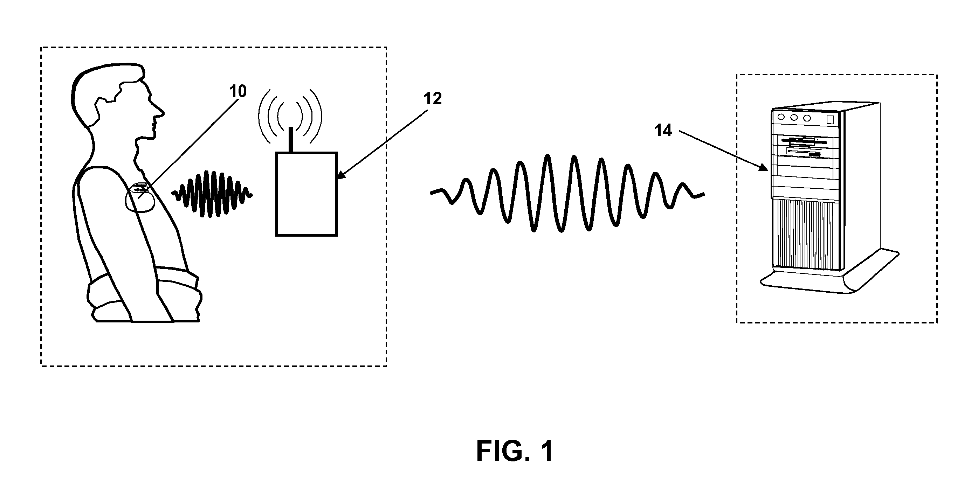

Fig. 1 zeigt ein implantierbares medizinisches Gerät in Form eines Herzschrittmachers (10) sowie ein externes Gerät (12), auch Patientengerät genannt, das in an sich bekannter Weise zumindest zeitweise über entsprechende drahtlose Datenkommunikationsschnittstellen mit dem Herzschrittmacher (10) verbunden ist. Das externe Gerät (12) ist darüber hinaus wenigstens zeitweise mit einem zentralen Servicecenter (14) verbunden. Die in Fig. 1 dargestellte Konstellation erlaubt es, dass vom Herzschrittmacher (10) aufgenommene Daten sowohl im Herzschrittmacher (10) selbst als auch beispielsweise im externen Gerät (12) oder im zentralen Servicecenter (14) bearbeitet werden können.

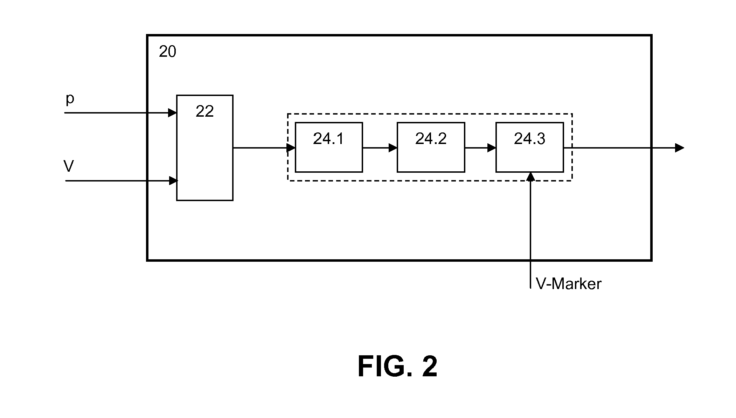

Fig. 2 zeigt einen erfindungsgemäßen Herzmonitor (20), der Bestandteil des Herzschrittmachers (10) aus Fig. 1 sein kann. Der Herzmonitor (20) besitzt einen Signaleingang zur Entgegennahme von Druck- oder Volumendaten oder Ersatzgrößen eines Herzens. Diese bilden ein Eingangssignal des Herzmonitors (20). Der Signaleingang kann mit weiteren Komponenten des Herzschrittmachers (10) verbunden sein oder auch direkt mit geeigneten Messsensoren, wie beispielsweise Druck- oder Kraftsensoren. Vorzugsweise ist der Herzschrittmacher in an sich bekannter Weise dazu ausgebildet, ein ventrikuläres Volumen durch eine intraventrikuläre Impedanzmessung zu bestimmen. Die im Inneren eines Ventrikels eines Herzens zu messende Impedanz wird maßgeblich von dem durch den Ventrikel eingeschlossenen Blutvolumen bestimmt, da das Blut eine höhere Leitfähigkeit besitzt als das umgebende Herzgewebe (Myokard). Die Messungen der intrakardialen und insbesondere der intraventrikulären Impedanz sowie die Auswertung des Impedanzverlaufs zum Bestimmen eines ventrikulären Volumens sind grundsätzlich bekannt und brauchen daher hier nicht näher beschrieben zu werden.

Messgrößen, die einen im Ventrikel herrschenden Druck repräsentieren, können beispielsweise mittels eines im Ventrikel angeordneten Druckmesssensors aufgenommen werden. Dieser kann, wie eingangs bereits angedeutet, Bestandteil einer ventrikulären Elektrodenleitung des Herzschrittmachers (10) sein. Der Drucksensor kann auch ein selbständiger Sensor sein, der ausgebildet ist, Druckmesswerte drahtlos an den Signaleingang des Herzmonitors (20) zu übermitteln. Der Druckmesssensor kann dabei beispielsweise passiv nach Art eines Transponders wirken. Weitere Methoden und Sensoren zur Bestimmung des im Ventrikel herrschenden Drucks sind eingangs angedeutet.

Der Signaleingang (22) des Herzmonitors (20) besitzt im hier dargestellten Ausführungsbeispiel zwei Eingänge, nämlich einen Eingang für Druckmesswerte oder äquivalente Größen und einen zweiten Eingang für Volumenmesswerte oder äquivalente Größen. Hieraus ergibt sich ein zweikanaliges Eingangssignal, dessen Signalwerte jeweils von einem Wertepaar gebildet wird von denen ein Wert einen Druckmesswert repräsentiert und der andere Wert einen Volumenmesswert repräsentiert. Die Wertepaare werden in Ihrer zeitlichen Reihenfolge nummeriert.

Mit diesem Signaleingang (22) des Herzmonitors (20) ist eine Auswerteeinheit (24) verbunden, die, wie in Fig. 2 angedeutet, dreistufig aufgebaut ist. Eine erste Stufe der Auswerteeinheit (24) ist dazu ausgebildet, das Eingangssignal, bestehend aus Signalwertepaaren derart zu segmentieren, dass jedes Segment des Eingangssignals einen Herzzyklus widerspiegelt. Dies geschieht im hier dargestellten Ausführungsbeispiel dadurch, dass die erste Stufe (24.1) der Auswerteeinheit (24) mit einem Markersignaleingang verbunden ist, der mit einem an sich bekannten Markerkanal des Herzschrittmachers (10) verbunden ist. Über den Markersignaleingang (26) kann die erste Stufe (24.1) der Auswerteeinheit (24) Markersignale empfangen, die das Auftreten und den jeweiligen Zeitpunkt eines kardialen Ereignisses, beispielsweise einer R-Zacke, repräsentieren. Derartige Markersignale sind grundsätzlich bekannt und brauchen hier nicht näher erläutert werden. Anhand des durch das Markersignal jeweils repräsentierten pro Herzzyklus einmaligen Ereignisses segmentiert die erste Stufe (24.1) der Auswerteeinheit (24) das Eingangssignal. Jedes so gewonnene Segment des Eingangssignals repräsentiert einen Herzzyklus und repräsentiert außerdem ein pV-Diagramm, das den Verlauf des Drucks im Ventrikel über den Verlauf des Volumens im Ventrikel repräsentiert. Beispielhafte Diagramme sind in Fig. 3 abgebildet.

Die Auswerteeinheit (24) führt die eingangs beschriebene Analyse der Umlaufrichtung des durch die Signalwertepaare repräsentierten pV-Diagramms durch, indem sie ein zweidimensionales Koordinatensystem mit einem Ursprung im Zentrum eines jeweiligen pV-Diagramms generiert und für jedes Signalwertepaar einen Vektor vom Mittelpunkt des Koordinatensystems zu einem jeweiligen Signalwertepaar bestimmt um die Umlaufrichtung aufeinanderfolgender Signalwertepaare zu bestimmen. Diese sollte monoton in eine Richtung verlaufen. Falls einzelne Signalwertepaare eine Umkehrung der Umlaufrichtung bewirken, werden diese Signalwertepaare eliminiert bzw. korrigiert (siehe Seite 3,17 - Seite 4,14). Eben diesen Auswertungen des Eingangssignals durch die zweite Auswertungsstufe (24.2) der Auswerteeinheit (24) kann die zweite Auswertungsstufe (24.2) mit der Auswerteeinheit 24 auch die weiteren eingangs beschriebenen Eigenschaften besitzen, die zu einer Vorauswertung einer jeweiligen Sequenz des Eingangssignals führen, also beispielsweise dazu ausgebildet sein, Kreuzungspunkte der durch die Signalwertepaare repräsentierten pV-Diagramme zu erkennen und zu beheben.

Die Auswerteeinheit (24) besitzt außerdem eine zweite Auswertestufe (24.2), die jedes Segment des Eingangssignals in dem Sinne vorauswertet, wie dies eingangs beschrieben ist, indem die zweite Auswertestufe (24.2) der Auswertungseinheit (24) jedes Segment des Eingangssignals daraufhin überprüft, ob das jeweilige Segment des Eingangssignals ein pV-Diagramm repräsentiert, für das die abgeleiteten Qualitätsmerkmale den Qualitätskriterien entsprechen. Dafür ist die zweite Auswertungsstufe (24.2) der Auswerteeinheit (24) dazu ausgebildet, ein von ihr generiertes korrigiertes Eingangssignalsegment mit dem jeweils ursprünglichen Segment des Eingangssignals zu vergleichen (Morphologie, Fläche, Verhältnis Umfang zu Fläche, Abstand zwischen Anfangs und Endpunkt oder Anzahl der Messwerte) und in Abhängigkeit eines jeweiligen Vergleichsergebnisses zu entscheiden, ob sich das korrigierte pV-Diagramm für eine weitere Auswertung eignet oder nicht.

Eine dritte Auswertungsstufe (24.3) der Auswerteeinheit (24) ist schließlich dazu ausgebildet, die von der zweiten Auswertungsstufe (24.2) generierten korrigierten Eingangssignalsegmente derart zu verarbeiten, dass die dritte Auswertungsstufe (24.3) aus diesen Eingangssignalsegmenten jeweils einen Wert (28) für einen oder mehrere der folgenden Parameter ableitet:
- Kontraktilitätsindex (Emax = ESP/(ESV - Vo)): Emax entspricht dem Anstieg jener Geraden die durch Vo gehend die pV-Kurve am linken oberen Rand gerade noch berührt (siehe Fig. 3).
- End-diastolisches Volumen (EDV): Das maximale Blutvolumen im Ventrikel, gemessen am Ende der Füllphase jedes Herzschlages
- End-systolisches Volumen (ESV): Das minimale Blutvolumen im Ventrikel, gemessen am Ende der Austreibungsphase
- Schlagvolumen (SV): Das Schlagvolumen eines einzelnen Herzschlages (SV = EDV - ESV)
- Ejektionsfraktion (EF = SV/EDV)
- Effektive arterielle Elastanz (Ea): Die effektive Elastanz des an den linken Ventrikel anschließenden arteriellen Systems (Ea = ESP/SV)
- Externe Arbeit (EW): Jener Anteil der Herzarbeit, die direkt in mechanische Energie umgewandelt wird. Bestimmt wird dieser Parameter durch die Berechnung der Fläche innerhalb der PV-Kurve;
- Potentielle Energie (PE): Jener Anteil der Herzarbeit, die nicht in mechanische Energie umgewandelt werden kann. PE bezeichnet eine Fläche, die an die PV-Kurve linksseitig anschließt. Sie verläuft ausgehend vom Residualvolumen (Vo) über jene Gerade die Vo mit dem End-systolischen Druck verbindet, weiter dem Verlauf der isovolumetrischen Relaxation folgend, bis zum Öffnen der Einstromklappe und wieder zurück zu Vo (siehe Fig. 3). Möglichkeiten zur direkten Bestimmung von Vo sind durch die Veränderung von Elektrostimulationsparametern wie AV und VV Verzögerung, Herzrate aber auch durch die künstliche Erzeugung von Extrasystolen gegeben da die verschiedene End-systolischen Drücke auf einer Linie liegen, deren Schnittpunkt mit der Volumenachse, Vo entspricht. Bei der Verwendung von Ersatzgrößen werden diese Merkmale entsprechend transformiert. Vo wird zu diesem Zweck geschätzt oder aus echokardiographischen Vermessungen bestimmt.
- Druck-Volumen Fläche (PVA): Der Gesamte Energiebedarf des Herzens während eines Schlages (PVA = PE + EW). Diese Größe kann zur Bestimmung des myokardialen Sauerstoffbedarfs verwendet werden;
- EW/PE: Die Effizienz des Herzens kann aus dem Verhältnis von EW und PE bestimmt werden. Je größer dieses Verhältnis ist umso höher ist der Anteil jener Energie die vom Herzen in mechanische Arbeit umgesetzt werden kann. Als Alternative wird für den gleichen Zweck auch EW/PVA, also das Verhältnis von geleisteter mechanischer Arbeit zum Gesamten Energiebedarf des Herzens, verwendet;
- Arteriell-ventrikuläre-Kopplung (AVC): AVC = Ea/Emax = (ESV-Vo)/ SV. Dieser Parameter kann auch ohne Messung des linksventrikulären Drucks bestimmt werden.
- Preload-Recruitable-Stroke_Work (PRSW): Beschreibt das Verhältnis von EW und EDV und dient als Vor- und Nachlast unabhängiger Parameter für die myokardiale Kontraktilität (alternative zu Emax).

Einige dieser Parameter sind in den in Fig. 3 beispielhaft abgebildeten pV-Diagrammen jeweils noch einmal bildlich erläutert.

Die Auswerteeinheit (24) und insbesondere deren dritte Auswertestufe (24.3) ist schließlich mit einer Steuereinheit und/oder wenigstens einer Stimulationseinheit des Herzschrittmachers (10) derart verbunden, dass die Auswerteeinheit (24) die Abgabe von Stimulationsimpulsen durch den Herzschrittmacher (10) über dessen Elektrodenleitungen beeinflussen kann.

Wie grundsätzlich bekannt, wirkt der Herzschrittmacher (10) als Herzstimulator und ist als solcher ausgebildet, über einen Herzstimulator angeschlossene Elektrodenleitungen elektrische Stimulationsimpulse in an sich bekannter Art und Weise an das Myokard einer jeweiligen Kammer (Ventrikel oder Atrium) eines Herzens abzugeben, um auf diese Weise eine stimulierte Kontraktion der jeweiligen Herzkammer auszulösen.

Das Verhalten eines stimulierten Herzens hängt maßgeblich davon ab, zu welchem Zeitpunkt Stimulationsimpulse an die verschiedenen Kammern eines Herzens abgegeben werden. Hierfür maßgebliche Zeitintervalle in der Steuerung eines Herzstimulators sind eine atrioventrikuläre Verzögerungszeit (atrioventricular delay AVD) und eine interventrikuläre Verzögerungszeit (VVD), die im Falle des in Fig. 1 abgebildeten Herzschrittmachers (10) durch die Auswerteeinheit (24) des Herzmonitors (20) zu beeinflussen sind.

Konkret ist die Auswerteeinheit (24) dazu ausgebildet, die Dauer einer jeweiligen atrioventrikulären Verzögerungszeit und einer jeweiligen interventrikulären Verzögerungszeit zu variieren, um zum einen auf diese Weise das Residualvolumen Vo zu bestimmen, wie es zur Bestimmung des Kontraktilitätsindex (Emax) erforderlich ist. Alternativ kann zur Bestimmung des Residualvolumens die Herzfrequenz gezielt moduliert werden, um eine Änderung des Füllzustandes zu Beginn der Systole und damit eine Preloadvariation zu realisieren.

Zum anderen ist die Auswerteeinheit (24) dazu ausgebildet, die atrioventrikuläre Verzögerungszeit und/oder die interventrikuläre Verzögerungszeit und/oder die Stimulationsfrequenz zu variieren, um auf diese Weise für die atrioventrikuläre Verzögerungszeit und/oder die interventrikuläre Verzögerungszeit und/oder die Stimulationsfrequenz aufeinander abgestimmt optimierte Größen zu ermitteln, für die sich ein globales Optimum der von der dritten Auswertungsstufe (24.3) aus dem Druck-Volumendiagramm abgeleiteten Größen ergibt.

Wie bereits angedeutet können die verschiedenen Bestandteile des Herzmonitors (20) auf verschiedene physische Entitäten, beispielsweise den Herzschrittmacher (10) und das externe oder Patientengerät (12) oder auch das zentrale Servicecenter (14), verteilt sein.

Bevorzugt ist jedoch, dass der Herzmonitor (20) als Ganzes Bestandteil des implantierbaren Herzschrittmachers (10) ist. Die von der dritten Auswertungsstufe (24.3) des Herzmonitors (20) generierten und aus dem Eingangssignal abgeleiteten Parameterwerte stehen außerdem an einem Ausgang (28) der dritten Auswertungsstufe (24.3) der Auswerteeinheit (24) bereit. Dieser Ausgang (28) ist vorzugsweise mit einem Speicher und/oder einer Telemetrieeinheit des Herzschrittmachers (10) verbunden, um die abgeleiteten Parameterwerte auf diese Weise telemetrisch zum Patientengerät (12) und von dort aus weiter zum zentralen Servicecenter (14) übertragen zu können. Das zentrale Servicecenter (14) kann dann dazu ausgebildet sein, aus den empfangenen abgeleiteten Parameterwerten weitere Werte, beispielsweise Trends, die die Entwicklung der Parameterwerte widerspiegeln, abzuleiten und einem behandelnden Arzt zuzuleiten oder zur Verfügung zu stellen.

Auf diese Weise kann der Herzmonitor (20) dazu benutzt werden, beispielsweise die Wirksamkeit einer kardialen Resynchronisationstherapie (CRT) zu überwachen oder ein besseres Monitoring eines Langzeitverlaufs unter Verwendung klinisch anerkannter Parameter durchzuführen.

Es ergeben sich in der Folge erweiterte Optimierungsmöglichkeiten der Implantatparameter (also der Einstellgrößen des Herzschrittmachers) durch eine genauere Bestimmung eines aktuellen Herzstatus. Außerdem sind mit Hilfe der erfindungsgemäß gewonnenen pV-Diagramme auch Rückschlüsse auf das periphere System möglich.

## Patentansprüche

1. Herzmonitor, der mit wenigstens einem Sensor für Druck- und Volumendaten oder Ersatzgrößen eines Herzens verbunden oder zu verbinden ist und der eine Auswerteeinheit zur Verarbeitung der den zeitlichen Verlauf von Druck- und Volumendaten oder Ersatzgrößen des Herzens widerspiegelnden Eingangssignale aufweist, die derart ausgebildet ist, dass die Auswerteeinheit eine Segmentierung der Eingangssignale entsprechend einzelner, abgeschlossener Herzzyklen vornimmt und so gewonnene Segmente der Eingangssignale jeweils daraufhin überprüft, ob ein jeweiliges Segment der Eingangssignale ein pV-Diagramm repräsentiert, das vorgegebenen Qualitätsbedingungen entspricht, wobei die Auswerteeinheit ausgebildet ist, aufeinander folgende Signalwertepaare der Eingangssignale hinsichtlich einer Umlaufrichtung eines durch die Signalwertepaare repräsentierten pV-Diagramms auszuwerten, **dadurch gekennzeichnet, dass** die Auswerteeinheit ausgebildet ist, die Umlaufrichtung eines jeweiligen durch Signalwertepaare repräsentierten pV-Diagramms dazu zu verwenden, ungültige Signalwertepaare zu erkennen und gegebenenfalls zu korrigieren, um ein korrigiertes pV-Diagramm zu erstellen.

2. Herzmonitor nach Anspruch 1, **dadurch gekennzeichnet, dass** die Auswerteeinheit ausgebildet ist, eine Umlaufrichtung eines jeweils durch die Signalwertepaare repräsentierten pV-Diagramms durch Auswerten der Richtung zweidimensionaler Vektoren zu bestimmen, indem ein Mittelpunkt eines zweidimensionalen Koordinatensystems mit einem Ursprung im Zentrum eines jeweiligen pV-Diagramms generiert und für jedes Signalwertepaar einen Vektor vom Mittelpunkt des Koordinatensystems zu einem jeweiligen Signalwertepaar bestimmt um die Umlaufrichtung aufeinander folgender Signalwertepaare zu bestimmen.

3. Herzmonitor nach Anspruch 1, **dadurch gekennzeichnet, dass** die Auswerteeinheit ausgebildet ist, Signalwertepaare zu verwerfen, die eine abweichende Umlaufrichtung haben, um ein korrigiertes pV-Diagramm zu erstellen.

4. Herzmonitor nach einem der Ansprüche 1 bis 3 mit einem Markersignaleingang für Markersignale, die definierte Ereignisse innerhalb eines jeweiligen Herzzyklusses einschließlich des Zeitpunktes ihres Auftretens repräsentieren, **dadurch gekennzeichnet, dass** die Auswerteeinheit ausgebildet ist, eine Segmentierung der Eingangssignale anhand über den Markersignaleingang zu empfangender Markersignale vorzunehmen.

5. Herzmonitor nach einem der Ansprüche 1 bis 3, **dadurch gekennzeichnet, dass** die Auswerteeinheit ausgebildet ist, eine Segmentierung der Eingangssignale anhand morphologischer Merkmale der durch die Signalwertepaare des Eingangssignals repräsentierten pV-Diagramme vorzunehmen und/oder eine Approximation der durch die Signalwertepaare repräsentierten pV-Diagramme durch Ersatzfunktionen wie zum Beispiel Ellipsen, Rechteck, Trapez oder dergleichen vorzunehmen, um ein korrigiertes pV-Diagramm zu erhalten und/oder Kreuzungspunkte der durch die Signalwertepaare repräsentierten pV-Diagramme, die sich durch die Verbindung aufeinander folgender Signalwertepaare ergeben, automatisch zu erkennen und zu korrigieren, um ein korrigiertes pV-Diagramm zu erstellen und/oder um ein korrigiertes pV-Diagramm durch die Berechnung einer Hüllkurve zu erstellen die alle Signalwertepaare umschließt und/oder eine ein jeweiliges pV-Diagramm repräsentierende Folge von Signalwertepaaren durch eine oder mehrere parameteroptimierte Funktionen zu approximieren, um ein korrigiertes pV-Diagramm zu erhalten

6. Herzmonitor nach einem der Ansprüche 1 bis 3 mit einem EKG-Signaleingang für ein intrakardiales oder Oberflächen EKG-Signal, **dadurch gekennzeichnet, dass** die Auswerteeinheit ausgebildet ist, eine Segmentierung der Eingangssignale anhand eines über den EKG-Signaleingang zu empfangenden intrakardialen EKG-Signals vorzunehmen.

7. Herzmonitor nach Anspruch 5, **dadurch gekennzeichnet, dass** die Auswerteeinheit ausgebildet ist, eine jeweils größtmögliche Hüllkurve zu bestimmen, die kein Signalwertepaar eines ein jeweiliges pV-Diagramm repräsentierenden Signalsegmentes umhüllt, oder die kleinstmögliche Hüllkurve zu bestimmen, die die meisten Signalwertepaare eines ein jeweiliges pV-Diagramm repräsentierenden Signalsegmentes umhüllt.

8. Herzmonitor nach einem der Ansprüche 1, 5 oder 7, **dadurch gekennzeichnet, dass** die Auswerteeinheit ausgebildet ist, vor einer weiteren Auswertung korrigierte pV-Diagramme mit dem jeweiligen ursprünglichen, unkorrigierten pV-Diagramm zu vergleichen.

9. Herzmonitor nach Anspruch 8, **dadurch gekennzeichnet, dass** die Auswerteeinheit ausgebildet ist, den Vergleich anhand von Qualitätskriterien wie zum Beispiel der Anzahl der korrigierten oder verworfenen Signalwertepaare, Unterschiede in der Morphologie, Flächendifferenz, Verhältnis von Umfang zu Fläche, Schwerpunktverschiebung, Entfernung zwischen End und Startpunkt oder dergleichen durchzuführen und in Abhängigkeit eines jeweiligen Vergleichsergebnisses zu entscheiden, ob sich ein jeweiliges korrigiertes pV-Diagramm für eine weitere Auswertung eignet oder nicht.

10. Herzmonitor nach einem der Ansprüche 1 bis 9, **dadurch gekennzeichnet, dass** die Auswerteeinheit ausgebildet ist, im Rahmen einer weiteren Auswertung aus einem jeweiligen durch Signalwertepaare repräsentierten pV-Diagramm jeweils einen Wert für einen oder mehrere der folgenden oder ähnlichen Parametern abzuleiten:
- Kontraktilitätsindex (Emax)
- End-diastolisches Volumen (EDV)
- End-systolisches Volumen (ESV)
- Schlagvolumen (SV)
- Ejektionsfraktion (EF = SV/EDV);
- Effektive arterielle Elastanz (Ea)
- Externe Arbeit (EW)
- Potentielle Energie (PE)
- Druck-Volumen Fläche (PVA)
- Quotient aus externer Arbeit und potentieller Energie (EW/PE)
- Arteriell-ventrikuläre-Kopplung (AVC = Ea/Emax)
- End-systolischer Druck (ESP)
- Preload-Recruitable-Stroke_Work (PRSW)

11. System bestehend aus einem Herzmonitor nach Anspruch 10 und einem Herzstimulator, die miteinander verbunden sind, **dadurch gekennzeichnet, dass** der Herzmonitor ausgebildet ist die Abgabe von Stimulationsimpulsen durch den Herzstimulator zu beeinflussen:
- wobei die Auswerteeinheit ausgebildet ist, im Rahmen der weiteren Auswertung lokale Änderungen des Füllzustandes oder Kontraktilitätindex (Emax) durch eine Variation von Stimulationsparametern wie atrioventrikuläre Verzögerungszeit (AVD), interventrikuläre Verzögerungszeit (VVD), Herzzyklusdauer oder dergleichen anzuregen, um ein jeweiliges Residualvolumen Vo bestimmen zu können, oder
- wobei die Auswerteeinheit ausgebildet ist, eine der abgeleiteten Größen zur gleichzeitigen Optimierung von einer atrioventrikulären Verzögerungszeit (AVD) und/oder eine interventrikulären Verzögerungszeit (VVD) und/oder der Stimulationsfrequenz zu verwenden, indem die atrioventrikuläre Verzögerungszeit (AVD) und/oder die interventrikuläre Verzögerungszeit (VVD) und/oder die Simulationsfrequenz solange variiert werden, bis ein globales Optimum eines und oder mehrerer abgeleiteter Parameter erreicht ist.

12. Herzmonitor nach Anspruch 10, **dadurch gekennzeichnet, dass** die Auswerteeinheit ausgebildet ist, im Rahmen der weiteren Auswertung eine automatische Erkennung von transienten Änderungen des Kontraktilitätsindex (Emax), der Vorlast oder Nachlast zur Aktualisierung eines jeweiligen Wertes des Residualvolumens Vo zu verwenden.

## Claims

1. A heart monitor which is or can be connected at least to a sensor for pressure and volume data or substitute variable of a heart and which comprises an evaluation unit for processing input signals reflecting the course over time of pressure and volume data, or substitute variables of the heart, said evaluation unit being configured such that the evaluation unit segments the input signals in accordance with individual completed cardiac cycles and examines segments of the input signals obtained in this way as to whether a particular segment of the input signals represents a pV diagram, which corresponds to specified quality conditions, wherein the evaluation unit is configured to evaluate consecutive signal value pairs of the input signals in terms of a loop direction of a pV diagram represented by the signal value pairs, **characterised in that** the evaluation unit is configured to use the loop direction of a respective pV diagram represented by signal value pairs to identify and, where appropriate, to correct invalid signal value pairs in order to create a corrected pV diagram.

2. The heart monitor according to Claim 1, **characterised in that** the evaluation unit is configured to determine a loop direction of a respective pV diagram represented by the signal value pairs by evaluating the direction of two-dimensional vectors, **in that** it generates a midpoint of a two-dimensional coordinate system originating at the centre of a particular pV diagram and determines, for each signal value pair, a vector from the midpoint of the coordinate system to a particular signal value pair in order to determine the loop direction of consecutive signal value pairs.

3. The heart monitor according to Claim 1, **characterised in that** the evaluation unit is configured to reject signal value pairs that have a deviating loop direction in order to create a corrected pV diagram.

4. The heart monitor according to one of Claims 1 to 3, having a marker signal input for marker signals which represent defined events within a particular cardiac cycle, including the time at which they occurred, **characterised in that** the evaluation unit is configured to segment the input signals on the basis of marker signals to be received via the marker signal input.

5. The heart monitor according to one of Claims 1 to 3, **characterised in that** the evaluation unit is configured to segment the input signal on the basis of morphological characteristics of the pV diagrams represented by the signal value pairs of the input signal and/or to perform an approximation of the pV diagrams represented by the signal value pairs using substitute functions, such as ellipses, rectangles, trapezoids or the like, in order to obtain a corrected pV diagram and/or to automatically detect and correct points of intersection of the pV diagrams represented by the signal value pairs which are the result of the connection of consecutive signal value pairs in order to create a corrected pV diagram and/or in order to create a corrected pV diagram and/or in order to create a corrected pV diagram by the calculation of an envelope curve that encloses all signal value pairs and/or to approximate a sequence of signal value pairs representing a particular pV diagram with one or more parameter-optimised functions in order to obtain a corrected pV diagram.

6. The heart monitor according to one of Claims 1 to 3, with an ECG signal input for an intracardiac ECG signal or surface ECG, **characterised in that** the evaluation unit is configured to segment the input signals on the basis of an intracardiac ECG signal to be received via the ECG signal input.

7. The heart monitor according to Claim 5, **characterised in that** the evaluation unit is configured to determine a respective greatest possible envelope curve that does not enclose any signal value pair of a signal segment representing a particular pV diagram, or to determine the smallest possible envelope curve that encloses most signal value pairs of a signal segment representing a particular pV diagram.

8. The heart monitor according to one of Claims 1, 5 or 7, **characterised in that** the evaluation unit is configured to compare corrected pV diagrams with the respective original uncorrected pV diagram prior to a further evaluation.

9. The heart monitor according to Claim 8, **characterised in that** the evaluation unit is configured to conduct the comparison on the basis of quality criteria, for example such as the number of corrected or rejected signal value pairs, differences in the morphology, the area difference, ratio of circumference to area, focal point shift, the distance between end and starting point, or the like, and to compare and decide, as a function of a respective comparison result, whether or not a particular corrected pV diagram is suited for further evaluation.

10. The heart monitor according to one of Claims 1 to 9, **characterised in that** the evaluation unit is configured, within the scope of a further evaluation, to derive a value for one or more of the following or similar parameters from a particular pV diagram represented by signal value pairs:
- contractility index (Emax)
- end-diastolic volume (EDV)
- end-systolic volume (ESV)
- stroke volume (SV)
- ejection fraction (EF=SV/EDV);
- effective arterial elastance (Ea)
- external work (EW)
- potential energy (PE)
- pressure-volume area (PVA)
- quotient of external work and potential energy (EW/PE)
- arterial-ventricular coupling (AVC = Ea/Emax)
- end-systolic pressure (ESP)
- preload recruitable stroke work (PRSW)

11. A system consisting of a heart monitor according to Claim 10 and a heart stimulator that are connected to one another, **characterised in that** the heart monitor is configured to influence the output of stimulation pulses by the heart stimulator:
- wherein the evaluation unit is configured, within the scope of the further evaluation, to stimulate local changes of the fill state or contractility index (Emax) by varying stimulation parameters, such as the atrioventricular delay (AVD), interventricular delay (VVD), the cardiac cycle duration or the like, in order to be able to determine a particular residual volume Vo, or
- wherein the evaluation unit is configured to use one of the derived variables to simultaneously optimise an atrioventricular delay (AVD) and/or interventricular delay (VVD) and/or stimulation rate **in that** the atrioventricular delay (AVD) and/or interventricular delay (VDD) and/or stimulation rate are varied until a global optimum of one or more derived parameters is obtained.

12. The heart monitor according to Claim 10, **characterised in that** the evaluation unit is configured, within the scope of a further evaluation, to use an automatic identification of transient changes of the contractility index (Emax), the preload or afterload to update a particular value of the residual volume Vo.

## Revendications

1. Moniteur cardiaque relié ou destiné à être relié à au moins un capteur pour des données de pression ou de volume ou des valeurs de remplacement d'un coeur, et comportant une unité d'évaluation pour le traitement des signaux d'entrée reflétant l'évolution dans le temps de données de pression et de volume ou de valeurs de remplacement du coeur, conçue de telle façon que l'unité d'évaluation effectue une segmentation des signaux d'entrée en fonction de cycles cardiaques individuels terminés, et vérifie ensuite respectivement les segments de signaux d'entrée ainsi obtenus pour savoir si un segment respectif des signaux d'entrée représente un diagramme pV correspondant à des conditions qualitatives prédéfinies, l'unité d'évaluation étant conçue pour évaluer des paires de valeurs de signal consécutives des signaux d'entrée quant à une direction périphérique d'un diagramme pV représenté par les paires de valeurs de signal, **caractérisé en ce que** l'unité d'évaluation est conçue pour utiliser la direction périphérique d'un diagramme pV respectif représenté par des paires de valeurs de signal pour reconnaître des paires de valeurs de signal invalides et éventuellement les corriger, afin d'établir un diagramme pV corrigé.

2. Moniteur cardiaque selon la revendication 1, **caractérisé en ce que** l'unité d'évaluation est conçue pour déterminer une direction périphérique d'un diagramme pV respectivement représenté par les paires de valeurs de signal, par l'évaluation de la direction de vecteurs bidimensionnels, en générant un point central d'un système de coordonnées bidimensionnel avec une origine au centre d'un diagramme pV respectif et en déterminant, pour chaque paire de valeurs de signal, un vecteur allant du point central du système de coordonnées jusqu'à une paire de valeurs de signal respective, afin de déterminer la direction périphérique de paires de valeurs de signal consécutives.

3. Moniteur cardiaque selon la revendication 1, **caractérisé en ce que** l'unité d'évaluation est conçue pour rejeter des paires de valeurs de signal présentant une direction périphérique différente, afin d'établir un diagramme pV corrigé.

4. Moniteur cardiaque selon l'une des revendications 1 à 3, avec une entrée de signaux de marquage pour des signaux de marquage représentant des évènements définis au cours d'un cycle cardiaque respectif, y compris le moment de leur occurrence, **caractérisé en ce que** l'unité d'évaluation est conçue pour effectuer une segmentation des signaux d'entrée à l'aide de signaux de marquage à recevoir par le biais de l'entrée de signaux de marquage.

5. Moniteur cardiaque selon l'une des revendications 1 à 3, **caractérisé en ce que** l'unité d'évaluation est conçue pour effectuer une segmentation des signaux d'entrée à l'aide de caractéristiques morphologiques des diagrammes pV représentés par les paires de valeurs de signal du signal d'entrée, et/ou pour effectuer une approximation des diagramme pV représentés par les paires de valeurs de signal, par des fonctions de remplacement telles que par exemple des ellipses, un rectangle, un trapèze, afin d'obtenir un diagramme pV corrigé, et/ou pour reconnaître automatiquement et corriger des points de croisement entre les diagrammes pV représentés par les paires de valeurs de signal qui sont issus de l'assemblage de paires de valeurs de signal consécutives, afin d'établir un diagramme pV corrigé, et/ou pour établir un diagramme pV corrigé en calculant une enveloppe incluant toutes les paires de valeurs de signal, et/ou pour approximer une suite de paires de valeurs de signal représentant un diagramme pV respectif par une ou plusieurs fonctions à paramètres optimisés, afin d'obtenir un diagramme pV corrigé.

6. Moniteur cardiaque selon l'une des revendications 1 à 3, avec une entrée de signal d'ECG pour un signal d'ECG intracardiaque ou de surface, **caractérisé en ce que** l'unité d'évaluation est conçue pour effectuer une segmentation des signaux d'entrée à l'aide d'un signal d'ECG intracardiaque à recevoir par le biais de l'entrée de signal d'ECG.

7. Moniteur cardiaque selon la revendication 5, **caractérisé en ce que** l'unité d'évaluation est conçue pour déterminer respectivement une enveloppe la plus grande possible n'incluant aucune paire de valeurs de signal d'un segment de signal représentant un diagramme pV respectif, ou pour déterminer l'enveloppe la plus petite possible incluant la plupart des paires de valeurs de signal d'un segment de signal représentant un diagramme pV respectif.

8. Moniteur cardiaque selon l'une des revendications 1, 5 ou 7, **caractérisé en ce que** l'unité d'évaluation est conçue pour comparer des diagrammes pV corrigés avec le diagramme pV initial non corrigé correspondant, avant une nouvelle évaluation.

9. Moniteur cardiaque selon la revendication 8, **caractérisé en ce que** l'unité d'évaluation est conçue pour effectuer la comparaison à l'aide de critères qualitatifs, tels que par exemple le nombre de paires de valeurs de signal corrigées ou rejetées, des différences dans la morphologie, la différence de surface, le rapport entre le pourtour et la surface, le décalage de point de gravité, la distance entre le point final et le point de départ ou autres, et pour décider si un diagramme pV corrigé respectif est prêt ou non pour une nouvelle évaluation, en fonction d'un résultat de comparaison respectif.

10. Moniteur cardiaque selon l'une des revendications 1 à 9, **caractérisé en ce que** l'unité d'évaluation est conçue pour dériver une valeur pour un ou plusieurs des paramètres suivants ou similaires, dans le cadre d'une nouvelle évaluation, à partir d'un diagramme pV respectif représenté par des paires de valeurs de signal :
- l'indice de contractilité (Emax)
- le volume diastolique final (EDV)
- le volume systolique final (ESV)
- le volume de battement (SV)
- la fraction d'éjection (EF = SV/EDV) ;
- l'élastance artérielle effective (Ea)
- le travail externe (EW)
- l'énergie potentielle (PE)
- la surface pression-volume (PVA)
- le quotient du travail externe et de l'énergie potentielle (EW/PE)
- le couplage artériel-ventriculaire (AVC = Ea/Emax)
- la pression systolique finale (ESP)
- le Preload-Recruitable-Stroke_Work (PRSW).

11. Système constitué d'un moniteur cardiaque selon la revendication 10 et d'un stimulateur cardiaque reliés l'un à l'autre, **caractérisé en ce que** le moniteur cardiaque est conçu pour influencer la délivrance d'impulsions de stimulation par le stimulateur cardiaque :
- dans lequel l'unité d'évaluation est conçue pour stimuler, dans le cadre de la nouvelle évaluation, des modifications locales du niveau de remplissage ou de l'indice de contractilité (Emax) par une modification de paramètres de stimulation, tels que la temporisation auriculo-ventriculaire (AVD) la temporisation inter-ventriculaire (VVD), la durée du cycle cardiaque ou autres, afin de pouvoir déterminer un volume résiduel respectif, ou
- dans lequel l'unité d'évaluation est conçue pour utiliser l'une des valeurs dérivées pour l'optimisation simultanée d'une temporisation auriculo-ventriculaire (AVD) et/ou d'une temporisation inter-ventriculaire (VVD) et/ou de la fréquence de stimulation, en modifiant la temporisation auriculo-ventriculaire (AVD) et/ou la temporisation inter-ventriculaire (VVD) et/ou la fréquence de stimulation jusqu'à ce qu'un optimum global d'un ou de plusieurs paramètres dérivés soit atteint.

12. Moniteur cardiaque selon la revendication 10, **caractérisé en ce que** l'unité d'évaluation est conçu pour utiliser, dans le cadre de la nouvelle évaluation, une reconnaissance automatique de modifications transitoires de l'indice de contractilité (Emax), de la pré-charge ou de la post-charge, pour l'actualisation d'une valeur respective du volume résiduel Vo.
